# EUROPÄISCHE PATENTSCHRIFT

(11) **EP 0 393 725 B1**
(45) Veröffentlichungstag und Bekanntmachung des Hinweises auf die Patenterteilung: **13.12.1995**
(21) Anmeldenummer: 90109721.2
(22) Anmeldetag: 23.10.1986
(51) Int. Cl.: C07K 1/113

(54) **Verfahren zur Aktivierung von gentechnolgish hergestellten, heterologen, Disulfidbrücken aufweisenden eukaryontischen Proteinen nach Expression in Prokaryonten**
Process for activating genetically-engineered, heterologous eucaryotic proteins containing disulphide bridges after their expression in procaryotes
Procédé pour activer des protéines eucaryotiques hétérologues contenant des ponts disulfures et produites pour génie génétique, après expression dans des procaryotes

(30) Priorität: 23.10.1985 DE 3537708
(43) Veröffentlichungstag der Anmeldung: 24.10.1990
(62) Teilanmeldung aus: 86114731.2
(73) Patentinhaber: BOEHRINGER MANNHEIM GMBH, 68298 Mannheim (DE)
(72) Erfinder: Fischer, Stephan, Dr., D-8120 Weilheim (DE); Rudolf, Rainer, Dr., D-8400 Regensburg (DE); Mattes, Ralf, Dr., D-8125 Oberhausen (DE)
(74) Vertreter: Huber, Bernhard, Dipl.-Chem.

(56) Entgegenhaltungen:
- EP-A- 0 114 506
- WO-A-84/03711
- US-A- 4 432 895
- HOPPE-SEYLER'S Z. PHYSIOL. CHEM., Band 364, Juli 1983, Seiten 813-820, Walter de Gruyter & Co., Berlin, DE; R. RUDOLPH et al.: "Influence of glutathione on the reactivation of enzymes containing cysteine or cystine"
- BIOCHIMICA ET BIOPHYSICA ACTA, Band 787, 1984, Seiten 275-28o, Elsevier Science Publishers B.V., Amsterdam, NL; M. TREXLER et al.: "Residues Cys-1 and Cys-79 are not essential for refolding of reduced-denatured kringle 4 fragment of human plasminogen"
- PROCEEDINGS OF THE NATIONAL ACADEMY OF SCIENCES USA, Band 81, Juni 1984, Seiten 3273-3277, Washington, US; S. CABILLY et al.: "Generation of antibody activity from immunoglobulin polypeptide chains produced in Escherichia coli"
- JOURNAL OF BIOLOGICAL CHEMISTRY, Band 253, Nr. 10, 25. Mai 1978, Seiten 3452- 3458, Baltimore, US; G. ORSINI et al.: "The renaturation of reduced chymotrypsinogen A in guanidine HC1"
- Protein Folding, EPO Applied Technologies Series Vol. 12, Seiten v-vii (1993)

## Beschreibung

Die Erfindung betrifft ein Verfahren zur Aktivierung von gentechnologisch hergestellten, Disulfidbrücken enthaltenden eukaryontischen Proteinen nach Expression in Prokaryonten.

Bei der Expression von heterologen Proteinen in Prokaryonten bilden diese Proteine in den Wirtszellen oft inaktive, schwer lösliche Aggregate (sog. "refractile bodies"), die außerdem noch mit Proteinen der Wirtszellen verunreinigt sind. Es wird vermutet, daß die Bildung solcher "refractile bodies" eine Folge der bei der Expression entstehenden hohen Proteinkonzentration in der Zelle ist. Es ist bekannt, daß bei der Bildung von großen Enzymmengen in der Zelle die Aggregation der Enzyme zu unlöslichen, hochmolekularen, meist inaktiven Partikeln erfolgt. Bevor solche Proteine, z.B. für therapeutische Zwecke, verwendet werden können, müssen sie demzufolge gereinigt und in ihre aktive Form überführt werden.

Nach bekannten Verfahren kann eine Reaktivierung derartiger, als Aggregate vorliegender Proteine in mehreren Schritten erfolgen (vgl. z.B. R. Jaenicke, FEBS Federation of European Biochemical Societies, Vol. 52 (1979) 187 - 198; R. Rudolph et al., Biochemistry 18 (1979) 5572 - 5575).

Im ersten Schritt wird eine Solubilisierung durch Zugabe von starken Denaturierungsmitteln, beispielsweise Guanidin-Hydrochlorid oder Harnstoff in hoher Konzentration oder durch Zugabe von stark sauren Agentien, beispielsweise Glycin/Phosphorsäure-Mischungen erreicht. Als weitere Hilfsstoffe haben sich reduzierende SH-Reagentien (z.B. Dithioerythritol, DTE) und EDTA, beispielsweise bei der Renaturierung von LDH, bewährt. Sofern das Protein durch Proteine der Wirtszelle verunreinigt ist, schließt sich als nächster Schritt eine Reinigung mit an sich bekannten und üblichen Methoden, z.B. Gel- oder Ionenaustauschchromatographie, an. Anschließend wird stark verdünnt, damit die Konzentration des Denaturierungsmittels geringer wird. Bei Verwendung von Guanidin-Hydrochlorid wird dabei auf Werte unter 0,5 mol/l verdünnt. Bei Enzymen mit freien SH-Gruppen erwies sich die Zugabe von SH-Gruppen schützenden Agentien als vorteilhaft (vgl. z.B. R. Jaenicke, Journal Polymer Science, Part C 16 (1967) 2143 - 2160).

In der EP-A-0114506 werden Verfahren zur Isolierung, Reinigung und Reaktivierung einiger heterologer Expressionsprodukte aus Bakterienkulturen beschrieben; zur Reaktivierung werden die Lösungen der "refractile bodies" in einem starken Denaturierungsmittel a) direkt in eine Lösung in einem schwächeren Denaturierungsmittel überführt, die dann zur Rückbildung von Disulfidbrücken oxidierenden Bedingungen unterworfen wird; b) das Protein sulfoniert, dann in eine Lösung in einem schwachen Denaturierungsmittel überführt und die S-Sulfonatgruppen durch Behandlung mit einem Sulfhydrylreagens in seiner reduzierten und oxydierten Form, z.B. mit GSH/GSSG, in -S-S-Gruppen überführt; oder c) die Lösung in einem schwachen Denaturierungsmittel direkt mit dem Sulfhydryl-Reagens, z.B. mit GSH/GSSG, behandelt. Ein typisches Beispiel, bei dem die oben dargelegten Probleme auftreten, ist tPA.

Die Hauptkomponente der Proteinmatrix von geronnenem Blut ist polymeres Fibrin. Diese Proteinmatrix wird durch Plasmin gelöst, das aus Plaminogen über Aktivierung durch die sogenannten Plasminogen-Aktivatoren gebildet wird, z.B. durch tPA (Gewebs-Plasminogen-Aktivator, tissue-type plasminogen activator). Die enzymatische Aktivität von natürlichem oder aus Eukaryonten gentechnologisch gewonnenem tPA (katalytische Aktivierung von Plasminogen zu Plasmin) ist in Abwesenheit von Fibrin oder Fibrinspaltprodukten (FSP) sehr gering, kann aber in Gegenwart dieser Stimulatoren wesentlich gesteigert werden (um mehr als den Faktor 10). Diese sogenannte Stimulierbarkeit der Aktivität ist ein entscheidender Vorzug von tPA gegenüber anderen bekannten Plasminogenaktivatoren, wie Urokinase oder Streptokinase (vgl. z.B. M. Hoylaerts et al., J.Biol.Chem. 257 (1982) 2012-2029; Nieuwenhiuzen et al., Biochemica et Biophysica Acta 755 (1983) 531-533). Der Faktor der Stimulierbarkeit mit BrCN-Spaltprodukten wird daher in der Literatur verschiedentlich angegeben und mit bis zu 35 beziffert.

Ein tPA-artiges, nicht glycosyliertes Produkt wird auch in genetisch manipulierten Prokaryonten (nach Einschleusen der cDNA) gebildet; einem solchen Produkt kommt aber nicht die Stimulierbarkeit der Aktivität eines tPA aus Eukaryonten zu. Denkbar ist, daß dies darauf zurückzuführen ist, daß die Redoxbedingungen in der Prokaryontenzelle in solcher Weise von der Eukaryontenzelle, aus der das Gen stammt, verschieden sind, daß von Anfang an ein nicht aktives Produkt gebildet wird, was beispielsweise darauf zurückzuführen sein könnte, daß die zahlreichen SS-Brücken, die das natürliche aktive Molekül enthält, in falscher Weise verknüpft oder gar nicht gebildet sind. Für den therapeutischen Einsatz von tPA ist aber nicht nur die enzymatische Aktivität als solche erforderlich, sondern außerdem auch seine Stimulierbarkeit. Auf die Tatsache, daß die Prokaryontenzelle vermutlich nicht die richtigen Bedingungen schafft, um die Aktivität von Eukaryontenproteinen in der richtigen Weise auszubilden, wird für andere Substanzen in The EMBO Journal 4, Nr. 3 (1985) 775-780 hingewiesen.

Nach der EP-A-0093639 werden zur Reaktivierung von tPA die aus E.coli erhaltenen Zellpellets in 6 mol/l Guanidin-Hydrochlorid suspendiert, mit Ultraschall behandelt, inkubiert und anschließend vier Stunden gegen eine Lösung aus Tris-HCl (pH = 8,0), Natriumchlorid, EDTA und Tween 80 dialysiert. Nach Dialyse wird zentrifugiert, wobei im Überstand die Plasminogenaktivator-Aktivität zu finden ist. Auf diese Weise renaturierter tPA ist zwar proteolytisch aktiv, zeigt jedoch keine meßbare Stimulierbarkeit durch BrCN-Spaltprodukte (BrCN-FSP) von Fibirin, gemäß dem in J.H. Verheijen, Thromb. Haemostas., 48, (3), 260-269 (1982) beschriebenen Verfahren.

Orsini und Goldberg (J. Biol. Chem. 253 (1978), 3453-3458) beschreiben die Renaturierung von reduziertem Chymotrypsinogen A durch Überführung des denaturierten Proteins in einen Renaturierungspuffer, der GSH, GSSG und nicht denaturierende Konzentrationen von Guanidin-HCl bzw. Harnstoff enthält.

Für die Reaktivierung von denaturierten Proteinen ist aus dem Stand der Technik kein allgemein anwendbares Verfahren bekannt; dies gilt ganz besonders für tPA, weil das native Protein eine sehr komplexe Struktur besitzt; es enthält eine freie Thiolgruppe und 17 SS-Brücken, die theoretisch auf 2,2 x 10²⁰ verschiedene Weisen verknüpft werden können, wobei nur eine Struktur dem nativen Zustand entspricht. Die aus dem Stand der Technik bekannten Verfahren zur Reaktivierung von tPA führen zwar zu einem proteolytisch aktiven tPA, der aber keine meßbare Stimulierbarkeit zeigt; ein Aktivierungsverfahren, welches zu stimulierbarem tPA führt, ist nicht bekannt.

Aufgabe der vorliegenden Erfindung ist deshalb die Bereitstellung eines Verfahrens zur vollständigen Aktivierung von gentechnologisch hergestellten, heterologen, Disulfidbrücken enthaltenden eukaryontischen Proteinen nach Expression in Prokaryonten; diese Aufgabe wird mit dem Gegenstand der vorliegenden Erfindung gelöst.

Gegenstand der Erfindung ist ein Verfahren zur Aktivierung von gentechnologisch hergestellten, heterologen, Disulfidbrücken enthaltenden eukaryontischen Proteinen nach Expression in Prokaryonten durch Zellaufschluß, Solubilisierung unter denaturierenden und reduzierenden Bedingungen und Reaktivierung unter oxidierenden Bedingungen in Gegenwart von GSH/GSSG,
das dadurch gekennzeichnet ist, daß man in der Stufe der Reaktivierung bei einem pH-Wert von 8 bis 12, einer GSH-Konzentration von 0,1 bis 20 mmol/l, einer GSSG-Konzentration von 0,01 bis 3 mmol/l und mit einer nicht denaturierenden Konzentration des Denaturierungsmittels arbeitet und daß man in der Reaktivierungsstufe als Denaturierungsmittel Arginin und/oder wenigstens eine Verbindung der allgemeinen Formel R₂-C0-NRR₁ (I) verwendet, wobei R und R₁ H oder Alkyl mit 1 bis 4 C-Atomen und R₂ H oder NHR₁ oder Alkyl mit 1 bis 3 C-Atomen bedeutet, mit der Maßgabe, daß R und R₁ nicht gleichzeitig H sind.

Bevorzugte Ausgestaltungen dieses Verfahrens sind Gegenstand der Unteransprüche.

Als Denaturierungsmittel werden Arginin oder Harnstoffderivate gemäß Formel (I) verwendet.

Ferner können Gemische dieser Denaturierungsmittel verwendet werden. Vorzugsweise wird diese Aktivierungsstufe auch in Gegenwart eines Fremdproteins durchgeführt; als solches eignet sich in der Regel jedes Fremdprotein, solange es nicht proteolytisch wirksam ist; vorzugsweise wird Rinderserumalbumin (BSA), verwendet, z.B. in einer Menge von 1 bis 3 mg/ml. Der Zusatz von BSA bewirkt eine leichte Erhöhung der Ausbeute und Stabilisierung des Proteins (wahrscheinlich durch Schutz vor Oberflächendenaturierung und/oder proteolytischem Abbau).

Die übrigen Verfahrensbedingungen können den für Reaktivierungsstufen aus dem Stand der Technik bekannten und üblichen Bedingungen entsprechen. Die Dauer der Aktivierung (Inkubation) beträgt vorzugsweise 20 bis 48 Stunden bei Raumtemperatur. Die Halbwertszeit der Aktivierung liegt in Gegenwart von 0,5 mmol/l reduziertem (GSH) und oxidiertem (GSSG) Glutathion bei etwa 10 bis 15 Stunden bei 20°C. Bei einer längeren Inkubation (48 Stunden) unter Reoxidationsbedingungen nimmt die Stimulierbarkeit durch CNBr-FSP in der Regel ab. Die Aktivierungsstufe wird vorzugsweise in Gegenwart von EDTA durchgeführt, wobei die zweckmäßigste Konzentration ca. 1 mmol/l EDTA beträgt.

Die der Aktivierungsstufe (Reoxidation/Aktivierung) vorausgehenden und nachfolgenden Verfahrensschritte, wie Zellaufschluß, Solubilisierung (Solubilisierung/Reduktion) und gegebenenfalls eine oder mehrere der Aktivierungsstufe vorausgehende und/oder nachfolgende Reinigungsoperationen können nach aus dem Stand der Technik, z.B. aus der EP-A-0114506, EP-A-0093619, für derartige Verfahren bekannten und üblichen Methoden durchgeführt werden; für ein im Hinblick auf Ausbeute und Aktivierung optimales Ergebnis kann es aber zweckmäßig sein, einzelne oder alle Verfahrensschritte unter Berücksichtigung einer oder mehrerer der hier erläuterten Verfahrensausgestaltungen durchzuführen. Insbesondere ist es auch möglich, die erfindungsgemäße Stufe der Aktivierung in dem nach dem Aufschluß erhaltenen Gemisch ohne vorhergehende Denaturierung und/oder Reduktion durchzuführen, allerdings bei niedriger Ausbeute. Die Expression wird in Prokaryonten, vorzugsweise in P. putida, und insbesondere in E.coli, durchgeführt. Das erfindungsgemäße Verfahren ist jedoch genauso geeignet, wenn in anderen Prokaryonten (z.B. Bacilli) exprimiert wird.

Der Zellaufschluß kann durch hierfür übliche Methoden durchgeführt werden, z.B. mittels Ultraschall, Hochdruckdispersion oder Lysozym; er wird vorzugsweise in einer zur Einstellung eines neutralen bis schwach sauren pH-Wertes geeigneten Pufferlösung als Suspensionsmedium durchgeführt, wie z.B. in 0,1 mol/l Tris-HCl. Nach dem Zellaufschluß werden die unlöslichen Bestandteile ("refractile bodies") in beliebiger Weise abgetrennt, vorzugsweise durch Abzentrifugieren bei höheren g-Zahlen und längerer Zentrifugationszeit oder durch Filtration. Nach dem Waschen mit Agentien, die tPA nicht stören, fremde Zellproteine jedoch möglichst lösen, z.B. Wasser, Phosphat-Pufferlösung, gegebenenfalls unter Zusatz milder Detergentien wie Triton, wird der Niederschlag (Pellet) der Solubilisierung (Solubilisierung/Reduktion) unterworfen. Die Solubilisierung erfolgt vorzugsweise im alkalischen pH-Bereich, insbesondere bei pH = 8,6 ± 0,4 und in Gegenwart eines Reduktionsmittels aus der Mercaptangruppe und eines Denaturierungsmittels.

Als Denaturierungsmittel können die für Solubilisierungen aus dem Stand der Technik, z.B. aus der EP-A-0114506, bekannten und üblichen Denaturierungsmittel verwendet werden, und insbesondere Guanidin-Hydrochlorid oder Harnstoff. Die Konzentration an Guanidin-Hydrochlorid beträgt zweckmäßigerweise ca. 6 mol/l, die von Harnstoff ca. 8 mol/l. Ebenso können Verbindungen der allgemeinen Formel I eingesetzt werden.

Als Reduktionsmittel aus der Mercaptangruppe kann z.B. reduziertes Glutathion (GSH) oder 2-Mercaptoethanol verwendet werden, z.B. in einer Konzentration von ca. 50 bis 400 mmol/l und/oder insbesondere DTE (Dithioerythritol) bzw. DTT (Dithiothreitol), z.B. in einer Konzentration von ca. 80 bis 400 mmol/l. Die Solubilisierung erfolgt zweckmäßigerweise bei Raumtemperatur während einer Dauer (Inkubation) von 1 bis mehreren Stunden, vorzugsweise von zwei Stunden. Zur Verhinderung der Aufoxidation des Reduktionsmittels durch Luftsauerstoff kann es auch zweckmäßig sein, EDTA zuzusetzen. Neben der Solubilisierung/Reduktion hat die Solubilisierungsstufe auch einen Reinigungseffekt, da ein Großteil von mit tPA immunologisch nicht kreuzreagierendem Material (Fremdproteine) nicht in Lösung geht.

Nach der Solubilisierung und vor der Aktivierungsstufe können an sich bekannte und übliche Reinigungsstufen eingeschoben werden; als Reinigungsmethoden kommen z.B. sterische Ausschlußchromatographie (SEC) (in Gegenwart von Guanidin-Hydrochlorid oder Harnstoff) oder Ionenaustauscher (in Gegenwart von Harnstoff oder deren Derivate) in Frage; eine unspezifische Reoxidation kann durch Zusatz eines Reduktionsmittels (z.B. 2-Mercaptoethanol) oder durch pH-Werte 4,5 verhindert werden (vgl. z.B. R. Rudolph, Biochem. Soc. Transactions 13 (1985) 308-311). Wenn in der vorausgehenden Solubilisierungsstufe DTE verwendet wurde, muß dieses in einer Reinigungsstufe abgetrennt werden. Die Reinigung kann z.B. durch SEC über Sephadex G 100 in Gegenwart von Guanidin-Hydrochlorid und eines Reduktionsmittels, z.B. von GSH bei einem pH von 1 bis 4 erfolgen (bei diesem Schritt kann eine große Menge des Fremdproteins abgetrennt werden); oder durch Abtrennung der Denaturierungs-/Reduktionsmittel durch Entsalzen über Sephadex G 25 in 0,01 mol/l HCl bzw. 0,1 mol/l Essigsäure. Die Abtrennung der Denaturierungs-/Reduktionsmittel ist alternativ durch Dialyse gegen die gleichen Lösungen möglich.

Ein weiterer Reinigungsschritt kann sich an die Reaktivierungsstufe anschließen; eine solche Reinigung erfolgt in der Regel mittels Dialyse oder auch einer anschließenden Isolierung des aktivierten tPA, beispielsweise durch Affinitätschromatographie, beispielsweise über Lys-Sepharose.

Erfindungsgemäß gelingt es, tPA aus Prokaryonten so zu aktivieren, daß nicht nur eine Aktivierung der normalen biologischen Aktivität erreicht, sondern darüberhinaus auch eine Stimulierbarkeit im oben definierten Sinne erreicht wird, welche die Stimulierbarkeit des nativen tPA weit übersteigt und größer als Faktor 10 ist, ja sogar Faktor 50 übersteigen kann.

Ein weiteres eukaryontisches Protein, das erfindungsgemäß nach Expression in Prokaryonten aktiviert werden kann, ist β-Interferon.

Die nachfolgenden Beispiele erläutern die Erfindung näher, ohne sie darauf zu beschränken. Wenn nicht anders angegeben, beziehen sich Prozentangaben auf Gewichtsprozent und Temperaturangaben auf Grad Celsius.

### Beispiel 1

### a) Präparation der "refractile bodies"

100 g E.coli Zellfeuchtmasse, aufgenommen in 1,5 l 0,1 mol/l Tris/HCl (pH 6,5) und 20 mmol/l EDTA wurden homogenisiert (Ultra-Turrax, 10 Sek.) und 0,25 mg/ml Lysozym zugegeben. Nach 30 Min. Inkubation bei Raumtemperatur wurde erneut homogenisiert und auf 3°C abgekühlt. Der Zellaufschluß wurde durch Hochdruckdispersion (550 kg/cm²) erreicht. Anschließend wurde mit 300 ml 0,1 mol/l Tris/HCl (pH 6,5) und 20 mmol/l EDTA nachgespült. Nach Zentrifugation (2 Std. bei 27.000 g, 4°C) wurde das Pellet in 1,3 l 0,1 mol/l Tris/HCl (pH 6,5), 20 mmol/1 EDTA und 2,5 % Triton-x-100 aufgenommen und homogenisiert. Nach erneuter Zentrifugation (30 Min. bei 27.000 g, 4°C) wurde das Pellet in 1,3 l 0,1 mol/l Tris/HCl (pH 6,5), 20 mmol/l EDTA und 0,5 % Triton-x-100 aufgenommen und homogenisiert. Abwechselnde Zentrifugation (30 Min. bei 27.000 g, 4°C) und Homogenisation des Pellets in 1 l 0,1 mol/l Tris/HCl (pH 6,5) und 20 mmol/l EDTA wurde noch dreimal durchgeführt.

Der tPA-Gehalt der "refractile bodies" Präparationen wurde durch SDS-PAGE, Identifizierung der tPA-Banden durch "Western-blotting" und densitometrische Analyse quantifiziert. Die "refractile bodies" zeigen bei SDS-PAGE und "Wester-blotting" eine starke tPA-Bande mit einem Molekulargewicht von ca. 60 kDa. Der tPA-Anteil am Gesamtproteingehalt der "refractile bodies" beträgt ca. 21 %.

### b) Solubilisierung/Reduktion der "refractile bodies"

"Refractile bodies" wurden bei einer Proteinkonzentration von 1 bis 5 mg/ml in 0,1 mol/l Tris/HCl (pH 8,6), 6 mol/l Guanidin-Hydrochlorid, 0,15 bis 0,4 mol/l DTE und 1 mmol/l EDTA 2 bis 3 Std. bei Raumtemperatur inkubiert. Danach wurde unlösliches Material (Zellwandfragmente usw.) abzentrifugiert (z.B. 30 Min. bei 35.000 bis 50.000 g, 4°C). Der pH-Wert des Überstandes wurde mit konz. HCl auf pH 3 eingestellt. Denaturierungs- und Reduktionsmittel wurden dann durch Dialyse gegen 0,01 mol/l HCl bei 4°C abgetrennt.

### c) Reoxidation/Aktivierung

Reoxidation/Aktivierung erfolgte durch eine 1:50 bis 1:200 Verdünnung in 0,1 mol/l Tris/HCl (pH 10,5), 1 mmol/l EDTA, 1 mg/ml BSA, 0,5 mol/l L-Arginin, 2 mmol/l GSH, 0,2 mmol/l GSSG. Nach 17 bis 24 Stunden Aktivierung bei ca. 20°C wurde die Aktivität und im Vergleich zur Aktivität von nativem glykosyliertem tPA aus Eukaryonten die Ausbeute bestimmt.

Ausbeute bezogen auf den Gesamtproteingehalt der "refractile bodies": 2,5 ± 0,5 %
Stimulierbarkeit: 10 ± 5
Ausbeute bezogen auf den tPA-Anteil der "refractile bodies": ca. 12 %.

### d) Reoxidation/Aktivierung ohne Abtrennung der Denaturierungs-/Reduktionsmittel

"Refractile bodies" wurden bei einer Proteinkonzentration von 1,25 mg/ml in 0,1 mol/l Tris/HCl (pH 8,6), 6 mol/l Guanidin-Hydrochlorid, 0,2 mol/l DTE und 1 mmol/l EDTA 2 Stunden bei Raumtemperatur inkubiert. Danach wurde sofort Reoxidation durch eine 1:100 Verdünnung in 0,1 mol/l Tris/HCl (pH 10,5), 1 mmol/l EDTA, 1 mg/ml BSA, 0,3 mol/l L-Arginin und den in der Tabelle angegebenen Mengen an GSSG eingeleitet. Zusätzlich befand sich im Aktivierungsansatz eine Restkonzentration von 0,06 mol/l Guanidin-Hydrochlorid und 2 mmol/l DTE.

Abhängigkeit der Aktivierungsausbeute von der GSSG-Konzentration bei Aktivierung ohne Abtrennung der Denaturierungs-/Reduktionsmittel.

| GSSG (mmol/l) | Ausbeute' (%) | Stimulierbarkeit (Faktor) |
|---|---|---|
| 0,2 | 0 | - |
| 1 | 0,13 | 4,0 |
| 5 | 1,49 | 7,4 |
| 6 | 1,28 | 5,4 |
| 7 | 1,04 | 5,8 |
| 9 | 0,98 | 5,2 |
| 10 | 1,77 | 10,0 |
| 15 | 0 | - |
| 20 | 0 | - |

| | | |
|---|---|---|
| '= Ausbeute an aktivem tPA bezogen auf den Gesamtproteingehalt der "refractile bodies". | | |

### Beispiel 2

Eine RB ("refractile bodies")-Präparation (ca. 5 mg) wurde in 1 ml 0,1 mol/l Tris/HCl (pH = 8,6), 6 mol/l Guanidin-Hydrochlorid und 0,15 - 0,2 mol/l DTE 2 bis 3 Stunden bei Raumtemperatur inkubiert. Unlösliches Material (Zellwandfragmente usw.) wurde danach durch Zentrifugation (20 Minuten bei 17.000 g) abgetrennt. Denaturierungs- und Reduktionsmittel wurden durch Gelfiltration über Sephadex G 25 (superfine) in 0,01 mol/l HCl entfernt. Dabei wurde die Probe etwa um den Faktor 5 bis 10 verdünnt. Das reduzierte Material in 0,01 mol/l HCl wurde bei -20°C aufbewahrt.

### Beispiel 3

In den nachfolgenden Tabellen ist der Einfluß verschiedener erfindungsgemäßer Parameter auf die Aktivierung und Stimulierbarkeit von tPA zusammengestellt. Für diese Reoxidationsexperimente wurde das nach Beispiel 2 solubilisierte, reduzierte Protein nicht weiter vorgereinigt.

Das reduzierte Protein (in 0,01 mol/l HCl) wurde durch Verdünnung von 1:10 bis 1:500 in "Reoxidationspuffer" aktiviert. Die Aktivierung wurde nach 22 bis 48 Stunden Inkubation bei Raumtemperatur bestimmt. Die Aktivität des reoxidierten Proteins bezieht sich auf eine "Standard-Reoxidation" (= 100 %) in:
0,1 mol/l Tris/HCl (pH = 10,5) + 1 mmol/l EDTA
+ 0,5 mol/l L-Arginin
+ 1 mg/ml BSA
+ 0,5 mmol/l GSH (reduziertes Glutathion)
+ 0,5 mmol/l GSSG (Glutathiondisulfid).

Die Stimulierbarkeit errechnet sich aus ΔE_{+CNBrFSP}/ΔE_{-CNBrFSP} (vgl. W. Nieuwenhuizen et al., Biochimica et Biophysica Acta 755 (1983) 531-533). Die Aktivität (in Prozent) und die Stimulierbarkeit (Faktor) wurde nach J. H. Verheijen Thromb. Haemostas. 48(3), 266-269, (1982) bestimmt.

Es wurden folgende Ergebnisse erhalten:

### 1. Abhängigkeit der Aktivierungsausbeute vom Zusatz an L-Arginin oder Guanidin-Hydrochlorid

Reoxidation in 0,1 mol/l Tris/HCl (pH 10,5)
+ 1 mmol/l EDTA
+ 1 mg/ml BSA
+ 0,5 mmol/l GSH
+ 0,5 mmol/l GSSG
a) L-Arginin

| L-Arginin (mol/l) | Aktivität (%) | Stimulierbarkeit (Faktor) |
|---|---|---|
| 0 | 4 | 2,5 |
| 0,25 | 98 | 7,5 |
| 0,5 | 100 | 21,9 |
| 0,75 | 27 | 16,3 |
| 1,0 | 23 | 3,5 |

Bei diesem Experiment ist zu bedenken, daß tPA durch L-Arginin inhibiert wird. Der Abfall der Aktivierungsausbeute bei höheren L-Argininkonzentrationen ist deshalb bezüglich der Inhibition zu korrigieren.
b) Guanidin-Hydrochlorid (Gdn/HCl)

| (Gdn/HCl) (mol/l) | Aktivität (%) |
|---|---|
| 0 | 11 |
| 0,25 | 22 |
| 0,5 | 53 |
| 0,75 | 58 |
| 1,0 | 12 |

### 2. Abhängigkeit der Aktivierungsausbeute von Zusatz an Harnstoff und Harnstoffderivaten.

Reoxidation in 0,1 mol/l Tris (pH 10,5), 1 mmol/l EDTA, 1 mg/ml BSA, 5 mmol/l GSH, 0,2 mmol/l GSSG
a) Harnstoff

| Harnstoff (mol/l) | Aktivität (%) |
|---|---|
| 0 | 1 |
| 0,5 | 20 |
| 1 | 59 |
| 1,5 | 126 |
| 2 | 162 |
| 2,5 | 141 |
| 3 | 72 |
| 4 | 12 |
| 5 | 0 |

b) Methylharnstoff

| Methylharnstoff (mol/l) | Aktivität (%) |
|---|---|
| 0,5 | 22 |
| 1 | 174 |
| 1,5 | 313 |
| 2 | 375 |
| 2,5 | 332 |
| 3 | 215 |
| 4 | 12 |
| 5 | 0 |

c) Ethylharnstoff

| Ethylharnstoff (mol/l) | Aktivität (%) |
|---|---|
| 0,5 | 46 |
| 1 | 212 |
| 1,5 | 323 |
| 2 | 300 |
| 2,5 | 107 |
| 3 | 19 |
| 4 | 0 |
| 5 | 0 |

d) Dimethylharnstoff

| Dimethylharnstoff (mol/l) | Aktivität (%) | Stimulierbarkeit (Faktor) |
|---|---|---|
| 0,5 | 167 | 8,8 |
| 1 | 256 | 8,9 |
| 1,5 | 283 | 9,4 |
| 2 | 177 | 7,7 |
| 2,5 | 78 | 8,9 |
| 3 | 23 | 9,9 |
| 4 | 4 | 8,6 |
| 5 | 2 | 3,5 |

### 3. Abhängigkeit der Aktivierungsausbeute vom Zusatz an Fettsäureamiden:

Reoxidation in 0,1 mol/l Tris (pH 10,5), 1 mmol/l EDTA, 1 mg/ml BSA, 5 mmol/l GSH, 0,2 mmol/l GSSG.
a) Formamid

| Formamid (mol/l) | Aktivität (%) |
|---|---|
| 0 | 42 |
| 0,5 | 59 |
| 1 | 175 |
| 1,5 | 245 |
| 2 | 325 |
| 2,5 | 423 |
| 3 | 444 |
| 4 | 416 |
| 5 | 341 |

b) Methylformamid

| Methylformamid (mol/l) | Aktivität (%) |
|---|---|
| 0,5 | 100 |
| 1 | 135 |
| 1,5 | 304 |
| 2 | 389 |
| 2,5 | 466 |
| 3 | 452 |
| 4 | 425 |
| 5 | 121 |

c) Acetamid

| Acetamid (mol/l) | Aktivität (%) |
|---|---|
| 0,5 | 72 |
| 1 | 134 |
| 1,5 | 207 |
| 2 | 261 |
| 2,5 | 204 |
| 3 | 237 |
| 4 | 198 |
| 5 | 141 |

d) Propionamid

| Propionamid (mol/l) | Aktivität (%) |
|---|---|
| 0,5 | 95 |
| 1 | 99 |
| 1,5 | 197 |
| 2 | 150 |
| 2,5 | 101 |
| 3 | 39 |
| 4 | 2 |
| 5 | 0 |

e) Butyramid

| Butyramid (mol/l) | Aktivität (%) |
|---|---|
| 0,5 | 55 |
| 1 | 52 |
| 1,5 | 17 |
| 2 | 0 |

### 4. Abhängigkeit der Aktivierungsausbeute vom pH-Wert

Reoxidation in 0,1 mol/l Tris/HCl + 1 mmol/l EDTA
+ 0,5 mol/l L-Arginin
+ 1 mg/ml BSA
+ 0,5 mmol/l GSH
+ 0,5 mmol/l GSSG

| pH | Aktivität (%) | Stimulierbarkeit (Faktor) |
|---|---|---|
| 7 | 1 | - |
| 8 | 22 | 3,0 |
| 9 | 89 | 13,6 |
| 10 | 105 | 20,3 |
| 11 | 95 | 21,3 |

### 5. Abhängigkeit der Aktivierungsausbeute von der GSH/GSSG-Konzentration

Reoxidation in 0,1 mol/l Tris/HCl, pH 10,5,
+ 1 mmol/l EDTA
+ 0,5 mol/l L-Arginin
+ 1 mg/ml BSA
a) + 1 mmol/l GSH

| (GSSG) (mmol/l) | Aktivität (%) | Stimulierbarkeit (Faktor) |
|---|---|---|
| 0,1 | 239 | 14,9 |
| 0,2 | 273 | 15,3 |
| 0,5 | 193 | 13,3 |
| 1 | 198 | 12,5 |
| 5 | 17 | 2,1 |
| 10 | 0 | - |
| 20 | 0 | - |

b) + 0,2 mmol/l GSSG

| (GSH) (mmol/l) | Aktivität (%) | Stimulierbarkeit (Faktor) |
|---|---|---|
| 0,05 | 15 | 2,2 |
| 0,1 | 40 | 3,8 |
| 0,2 | 112 | 6,8 |
| 0,5 | 142 | 7,4 |
| 1 | 273 | 6,8 |
| 5 | 260 | 7,9 |
| 10 | 143 | 6,3 |
| 20 | 55 | 5,1 |

### 6. Abhängigkeit der Aktivierungsausbeute von der Protein-Konzentration bei der Reoxidation (Verdünnung 1:20 - 1:500)

Reoxidation in 0,1 mol/l Tris/HCl (pH 10,5)
+ 1 mmol/l EDTA
+ 0,5 mol/l L-Arginin
+ 1 mg/ml BSA
+ 0,5 mmol/l GSH
+ 0,5 mmol/l GSSG

| Verdünnung | Aktivität (%) | Stimulierbarkeit (Faktor) |
|---|---|---|
| 1:10 | 29 | 15,3 |
| 1:20 | 45 | 25,4 |
| 1:50 | 69 | 37,9 |
| 1:100 | 100 | 37,9 |
| 1:200 | 79 | 52,7 |
| 1:500 | 29 | 28,7 |

### 7. Abhängigkeit der Aktivierungsausbeute vom Zusatz an BSA

Reoxidation in 0,1 mol/l Tris/HCl (pH 10,5)
+ 1 mmol/l EDTA
+ 0,5 mol L-Arginin
+ 0,5 mmol/l GSH
+ 0,5 mmol/l GSSG

| BSA (mg/ml) | Aktivität (%) |
|---|---|
| 0 | 47 |
| 0,5 | 83 |
| 1 | 100 |
| 3 | 102 |
| 5 | 52 |

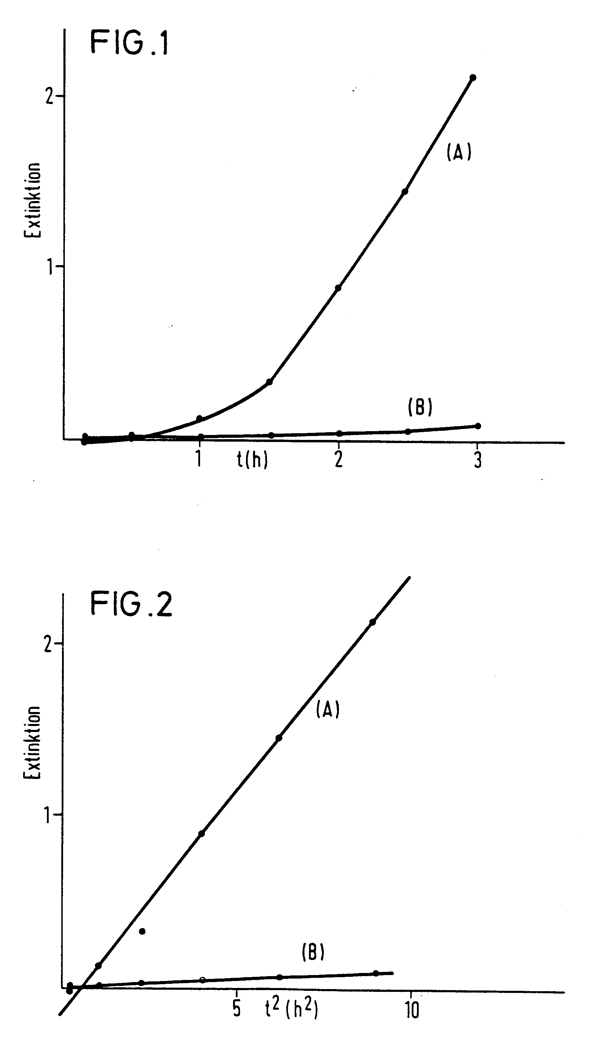

Die Figuren 1 und 2 zeigen die Aktivität mit und ohne CNBr-FSP im Standardtest nach 17 Stunden Reoxidation bei Raumtemperatur in 0,1 mol/l Tris/HCl (pH = 10,5) + 1 mmol/l EDTA + 0,5 mol L-Arginin + 1 mg/ml BSA + 0,5 mmol/l GSH + 0,5 mmol/l GSSG. In den Fig. 1 und 2 bedeuten die Kurven (A) die Aktivität in Gegenwart von CNBr-FSP, die Kurven (B) die Aktivität ohne CNBr-FSP.

### Beispiel 4

### Aktivierung von gentechnologisch hergestelltem Interferon-β.

"Refractile bodies" wurden nach den vorgenannten Methoden produziert. Die Reduktion/Solubilisierung der "refractile bodies" wurde wie folgt durchgeführt: Das Pellet wurde 3 Stunden bei 25°C in 10 ml 9,1 mol/l Tris/HCl, pH 8,6, 6 mol/l Gdn·HCl, 1 mmol/l EDTA und 0,2 mol/l DTE inkubiert und nach 30 Minuten Zentrifugation bei 4°C und 48.000 g der pH des Überstandes auf ca. 3 mit konzentrierter HCl eingestellt. Anschließend wurde eine Gelfiltration über Sephadex G25 F in 0,01 mol/l HCl durchgeführt.

Das Eluat wurde auf Leitfähigkeit, Proteinkonzentration und Reaktivierbarkeit untersucht.

Die Aktivierbarkeit des reoxidierten Proteins bezieht sich auf eine "Standardaktivierung" (= 100 %) in 0,1 mol/l Tris/HCl, pH 10,5, 1 mmol/l EDTA, 5 mmol/l GSH, 0,5 mmol/l GSSG und 0,25 mol/l L-Arginin.
a) Abhängigkeit der Aktivierungsausbeute zum Zusatz an L-Arginin
Das Eluat wurde 1:50 mit 0,1 mol/l Tris/HCl, pH 8,5, 1 mmol/l EDTA, 5 mmol/l GSH, 0,5 mmol/l GSSG verdünnt und 20 Stunden bei 0°C aktiviert.

| L-Arginin-Abhängigkeit der Aktivierung | |
|---|---|
| L-Arginin (mol/l) | Aktivität (%) |
| 0 | 8 |
| 0,25 | 8 |
| 0,5 | 15 |
| 0,75 | 15 |

b) Abhängigkeit der Aktivierungsausbeute vom Zusatz an Harnstoff
Die Aktivierungslösung entsprach der von Punkt a); jedoch wurde 17 Stunden bei 0°C aktiviert.

| Harnstoff-Abhängigkeit der Aktivierung | |
|---|---|
| Harnstoff (mol/l) | Aktivität (%) |
| 0 | 13 |
| 0,5 | 100 |
| 1 | 200 |
| 1,5 | 100 |

c) Abhängigkeit der Aktivierungsausbeute vom Zusatz an Formamid
Aktivierung wie in a); die Proben wurden nach 17 Stunden Aktivierung bei 0°C untersucht.

| Formamid-Abhängigkeit der Aktivierung | |
|---|---|
| Formamid (mol/l) | Aktivität |
| 0 | 13 |
| 1 | 13 |
| 2 | 13 |
| 3 | 0 |
| 4 | 0 |

d) Abhängigkeit der Aktivierungsausbeute vom Redoxpuffer
Das Eluat wurde 1:50 in 0,1 mol/l Tris/HCl, pH 8,5, 1 mmol/l EDTA und 0,25 mol/l L-Arginin verdünnt und die Proben nach 17 Stunden Aktivierung bei 0°C untersucht.

| GSH/GSSG-Abhängigkeit der Aktivierung | | |
|---|---|---|
| GSH (mmol/l) | GSSG (mmol/l) | Aktivität (%) |
| 1 | 0,5 | 6 |
| 5 | 0,5 | 13 |
| 10 | 0,5 | 25 |
| 20 | 0,5 | 25 |
| 5 | 0,1 | 13 |
| 5 | 0,5 | 13 |
| 5 | 1,0 | 13 |
| 5 | 5 | 6 |

e) Abhängigkeit der Aktivierungsausbeute vom Zusatz von BSA
Das Eluat wurde 1:50 in 0,1 mol/l Tris/HCl, pH 8,5, 1 mmol/l EDTA, 5 mmol/l GSH, 0,5 mmol/l GSSG und 0,25 mol/l L-Arginin verdünnt und nach 17 Stunden Aktivierung bei 0°C untersucht.

| BSA-Abhängigkeit der Aktivierung | |
|---|---|
| BSA (mg/ml) | Aktivität (%) |
| 0 | 13 |
| 1 | 13 |
| 2 | 25 |
| 5 | 13 |

f) Abhängigkeit der Aktivierungsausbeute vom pH
Das Eluat wurde 1:50 in 0,1 mol/l Tris/HCl, 1 mmol/l EDTA, 5 mmol/l GSH, 0,5 mmol/l GSSG und 0,25 mol/l L-Arginin verdünnt und nach 17 Stunden Aktivierung bei 0°C untersucht.

| pH-Abhängigkeit der Aktivierung | |
|---|---|
| pH | Aktivität (%) |
| 6,5 | 0 |
| 7,5 | 6 |
| 8,5 | 13 |
| 9,5 | 50 |
| 10,5 | 100 |

## Patentansprüche

1. Verfahren zur Aktivierung von gentechnologisch hergestellten, heterologen, Disulfidbrücken enthaltenden eukaryontischen Proteinen nach Expression in Prokaryonten durch Zellaufschluß, Solubilisierung unter denaturierenden und reduzierenden Bedingungen und Reaktivierung unter oxidierenden Bedingungen in Gegenwart von GSH/GSSG,
**dadurch gekennzeichnet,** daß man in der Stufe der Beaktivierung bei einem pH-Wert von 8 bis 12, einer GSH-Konzentration von 0,1 bis 20 mmol/l, einer GSSG-Konzentration von 0,01 bis 3 mmol/l und mit einer nicht denaturierenden Konzentration des Denaturierungsmittels arbeitet und daß man in der Reaktivierungsstufe als Denaturierungsmittel Arginin und/oder Wenigstens eine Verbindung der allgemeinen Formel R₂-C0-NRR₁ (I) verwendet, wobei R und R₁ H oder Alkyl mit 1 bis 4 C-Atomen und R₂ H oder NHR₁ oder Alkyl mit 1 bis 3 C-Atomen bedeutet, mit der Maßgabe, daß R und R₁ nicht gleichzeitig H sind.

2. Verfahren nach Anspruch 1,
**dadurch gekennzeichnet,** daß in der Reaktivierungsstufe der pH-Wert 9,5 bis 11 beträgt.

3. Verfahren nach einem der Ansprüche 1 und 2,
**dadurch gekennzeichnet,** daß in der Reaktivierungsstufe die GSH-Konzentration 0,2 bis 10 mmol/l und/oder die GSSG-Konzentration 0,05 bis 1 mmol/l beträgt.

4. Verfahren nach einem der vorhergehenden Ansprüche,
**dadurch gekennzeichnet,** daß man nach der Solubilisierung und vor der Reaktivierung eine Reinigungsstufe durchführt.

5. Verfahren nach einem der Ansprüche 1 bis 3,
**dadurch gekennzeichnet,** daß die Reaktivierung ohne vorherige Abtrennung der Denaturierungs-/Reduktionsmittel durchgeführt wird, wobei die Reaktionslösung nach Denaturierung/Reduktion mit Reaktivierungspuffer verdünnt wird und bei der folgenden Reaktivierung die GSSG-Konzentration die verbleibende Restkonzentration an DTE übersteigt.

6. Verfahren nach einem der Ansprüche 1 bis 5,
**dadurch gekennzeichnet,** daß man die Expression in E.coli oder P.putida durchführt.

7. Verfahren nach einem der Ansprüche 1-6,
**dadurch gekennzeichnet,** daß die Konzentration an Arginin 0,1 bis 1,0 mol/l, insbesondere 0,25 bis 0,8 mol/l beträgt.

8. Verfahren nach einem der Ansprüche 1-6,
**dadurch gekennzeichnet,** daß die Konzentration der Verbindung der allgemeinen Formel I 0,5 bis 4 mol/l, insbesondere 1 bis 3,5 mol/l beträgt.

9. Verfahren nach einem der vorhergehenden Ansprüche,
**dadurch gekennzeichnet,** daß man in der Stufe der Reaktivierung in Gegenwart eines nicht proteolytisch wirksamen Proteins, insbesondere in Gegenwart von Rinderserumalbumin, arbeitet.

10. Verfahren nach einem der vorhergehenden Ansprüche,
**dadurch gekennzeichnet,** daß man den Zellaufschluß mittels Ultraschall, Hochdruckdispersion oder Lysozym durchführt.

11. Verfahren nach Anspruch 10,
**dadurch gekennzeichnet,** daß man den Aufschluß in einer verdünnten wäßrigen Pufferlösung, insbesondere in 0,1 mol/l Tris, bei einem neutralen bis schwach sauren pH-Wert durchführt.

12. Verfahren nach einem der vorhergehenden Ansprüche,
**dadurch gekennzeichnet,** daß man nach dem Zellaufschluß die unlöslichen Bestandteile abtrennt.

13. Verfahren nach einem der vorhergehenden Ansprüche,
**dadurch gekennzeichnet,** daß man in der Stufe der Solubilisierung bei einem alkalischen pH-Wert in Gegenwart eines Reduktionsmittels aus der Mercaptogruppe und in Gegenwart eines Denaturierungsmittels arbeitet.

14. Verfahren nach Anspruch 13,
**dadurch gekennzeichnet,** daß man in Gegenwart einer
Verbindung der allgemeinen Formel I als Denaturierungsmittel arbeitet.

15. Verfahren nach Anspruch 14,
**dadurch gekennzeichnet,** daß die Konzentration der Verbindung der allgemeinen Formel I 8 mol/l beträgt.

16. Verfahren nach einem der Ansprüche 13 bis 15,
**dadurch gekennzeichnet,** daß man in Gegenwart von DTE, β-Mercaptoethanol, Cystein oder GSH arbeitet.

17. Verfahren nach einem der vorhergehenden Ansprüche,
**dadurch gekennzeichnet,** daß man Reinigung und Abtrennung von Reduktions-, Oxidations- oder Denaturierungsmitteln mittels sterischer Ausschlußchromatographie oder Dialyse durchführt.

18. Verfahren nach einem der vorhergehenden Ansprüche,
**dadurch gekennzeichnet,** daß man nach der Stufe der Reaktivierung eine Reinigungsstufe mittels Dialyse durchführt.

19. Verfahren nach einem der Ansprüche 1 bis 18,
**dadurch gekennzeichnet,** daß man als gentechnologisch hergestelltes eukaryontisches Protein tPA verwendet.

20. Verfahren nach einem der Ansprüche 1 bis 18,
**dadurch gekennzeichnet,** daß man als gentechnologisch hergestelltes eukaryontisches Protein Interferon β verwendet.

## Claims

1. Process for the activation of gene-technologically produced, heterologous, disulphide bridge-containing eukaryotic proteins after expression in prokaryotes by cell digestion, solubilisation under denaturing and reducing conditions and reactivation under oxidising conditions in the presence of GSH/GSSG, characterised in that, in the step of the reactivation, one works at a pH value of 8 to 12, a GSH concentration of 0.1 to 20 mmol/l, a GSSG concentration of 0.01 to 3 mmol/l and with a non-denaturing concentration of the denaturing agent and that, in the reactivation step, as denaturing agent, one uses arginine and/or at least one compound of the general formula R₂-CO-NRR₁ (I), whereby R and R₁ signify H or alkyl with 1 to 4 C-atoms and R₂ signifies H or NHR₁ or alkyl with 1 to 3 C-atoms, with the proviso that R and R₁ are not simultaneously H.

2. Process according to claim 1, characterised in that, in the reactivation step, the pH value amounts to 9.5 to 11.

3. Process according to one of claims 1 and 2, characterised in that, in the reactivation step, the GSH concentration amounts to 0.2 to 10 mmol/l and/or the GSSG concentration to 0.05 to 1 mmol/l.

4. Process according to one of the preceding claims, characterised in that one carries out a purification step after the solubilisation and before the reactivation.

5. Process according to one of claims 1 to 3, characterised in that the reactivation is carried out without previous separation of the denaturing/reducing agent, whereby the reaction solution is diluted after denaturing/reducing with reactivation buffer and, in the following reactivation, the GSSG concentration exceeds the remaining residual concentration of DTE.

6. Process according to one of claims 1 to 5, characterised in that one carries out the expression in E. coli or P. putida.

7. Process according to one of claims 1 - 6, characterised in that the concentration of arginine amounts to 0.1 to 1.0 mol/l, especially 0.25 to 0.8 mol/l.

8. Process according to one of claims 1 - 6, characterised in that the concentration of the compound of the general formula I amounts to 0.5 to 4 mol/l, especially 1 to 3.5 mol/l.

9. Process according to one of the preceding claims, characterised in that, in the step of reactivation, one works in the presence of a non-proteolytically effective protein, especially in the presence of bovine serum albumin.

10. Process according to one of the preceding claims, characterised in that one carries out the cell digestion by means of ultrasonics, high pressure dispersion or lysozyme.

11. Process according to claim 10, characterised in that one carries out the digestion in a dilute aqueous buffer solution, especially in 0.1 mol/l tris, at a neutral to weakly acidic pH value.

12. Process according to one of the preceding claims, characterised in that, after the cell digestion, one separates off the insoluble components.

13. Process according to one of the preceding claims, characterised in that, in the step of solubilisation, one works at an alkaline pH value in the presence of a reducing agent of the mercapto group and in the presence of a denaturing agent.

14. Process according to claim 13, characterised in that one works in the presence of a compound of the general formula I as denaturing agent.

15. Process according to claim 14, characterised in that the concentration of the compound of the general formula I amounts to 8 mol/l.

16. Process according to one of claims 13 to 15, characterised in that one works in the presence of DTE, β-mercaptoethanol, cysteine or GSH.

17. Process according to one of the preceding claims, characterised in that one carries out purification and separation of reducing, oxidising and denaturing agents by means of steric exclusion chromatography or dialysis.

18. Process according to one of the preceding claims, characterised in that, after the step of reactivation, one carries out a purification step by means of dialysis.

19. Process according to one of claims 1 to 18, characterised in that one uses t-PA as genetechnologically produced eukaryotic protein.

20. Process according to one of claims 1 to 18, characterised in that one uses interferon β as genetechnologically produced eukaryotic protein.

## Revendications

1. Procédé pour activer des protéines eucaryotiques hétérologues, contenant des ponts disulfure et préparées par génie génétique après expression dans des procaryotes par lyse des cellules, solubilisation dans des conditions dénaturantes et réductrices et réactivation dans des conditions oxydantes en présence de GSH/GSSG, caractérisé en ce que, dans l'étape de réactivation, on opère à un pH de 8 à 12, à une concentration en GSH de 0,1 à 20 mmol/l, à une concentration en GSSG de 0,01 à 3 mmol/l et avec une concentration non dénaturante de l'agent dénaturant et en ce que l'on utilise comme agent dénaturant dans l'étape de réactivation l'arginine et/ou au moins un composé de formule générale R₂-CO-NRR₁ (I) dans laquelle R et R₁ représentent H ou un alkyle de 1 à 4 atomes de carbone et R₂ représente H ou NHR₁ ou un alkyle de 1 à 3 atomes de carbone, à condition que R et R₁ ne soient pas simultanément H.

2. Procédé selon la revendication 1, caractérisé en ce que le pH est de 9,5 à 11 dans l'étape de réactivation.

3. Procédé selon l'une des revendications 1 et 2, caractérisé en ce que la concentration en GSH est de 0,2 à 10 mmol/l et/ou la concentration en GSSG est de 0,05 à 1 mmol/l dans l'étape de réactivation.

4. Procédé selon l'une des revendications précédentes, caractérisé en ce que l'on accomplit une étape de purification après la solubilisation et avant la réactivation.

5. Procédé selon l'une des revendications 1 à 3, caractérisé en ce que la réactivation est accomplie sans séparation préalable des agents dénaturants/réducteurs, la solution réactionnelle étant diluée avec un tampon de réactivation après la dénaturation/réduction et la concentration en GSSG dépassant la concentration résiduelle en DTE restante lors de la réactivation subséquente.

6. Procédé selon l'une des revendications 1 à 5, caractérisé en ce que l'on accomplit l'expression dans E. coli ou dans P. putida.

7. Procédé selon l'une des revendications 1 à 6, caractérisé en ce que la concentration en arginine est de 0,1 à 1,0 mol/l, en particulier de 0,25 à 0,8 mol/l.

8. Procédé selon l'une des revendications 1 à 6, caractérisé en ce que la concentration du composé de formule générale I est de 0,5 à 4 mol/l, en particulier de 1 à 3,5 mol/l.

9. Procédé selon l'une des revendications précédentes, caractérisé en ce que l'on opère en présence d'une protéine non active du point de vue protéolytique, en particulier en présence de sérumalbumine bovine, dans l'étape de réactivation.

10. Procédé selon l'une des revendications précédentes, caractérisé en ce que l'on accomplit la lyse des cellules à l'aide d'ultrasons, de dispersion sous haute pression ou de lysozyme.

11. Procédé selon la revendication 10, caractérisé en ce que l'on accomplit la lyse dans une solution tampon aqueuse diluée, en particulier dans tris 0,1 mol/l, à un pH neutre à faiblement acide.

12. Procédé selon l'une des revendications précédentes, caractérisé en ce que l'on sépare les constituants insolubles après la lyse des cellules.

13. Procédé selon l'une des revendications précédentes, caractérisé en ce que, dans l'étape de solubilisation, on opère à un pH alcalin en présence d'un réducteur du groupe des mercaptans et en présence d'un agent dénaturant.

14. Procédé selon la revendication 13, caractérisé en ce que l'on opère en présence d'un composé de formule générale I comme agent dénaturant.

15. Procédé selon la revendication 14, caractérisé en ce que la concentration du composé de formule générale I est de 8 mol/l.

16. Procédé selon l'une des revendications 13 à 15, caractérisé en ce que l'on opère en présence de DTE, de β-mercaptoéthanol, de cystéine ou de GSH.

17. Procédé selon l'une des revendications précédentes, caractérisé en ce que l'on accomplit la purification et la séparation des réducteurs, des oxydants ou des agents dénaturants par chromatographie d'exclusion stérique ou par dialyse.

18. Procédé selon l'une des revendications précédentes, caractérisé en ce que l'on accomplit une étape de purification par dialyse après l'étape de réactivation.

19. Procédé selon l'une des revendications 1 à 18, caractérisé en ce que l'on utilise le tPA comme protéine eucaryotique préparée par génie génétique.

20. Procédé selon l'une des revendications 1 à 18, caractérisé en ce que l'on utilise l'interféron β comme protéine eucaryotique préparée par génie génétique.
